Europäisches Patentamt

European Patent Office

Office européen des brevets

(19)

(11) Publication number: **0 174 706 B1**

# EUROPEAN PATENT SPECIFICATION

(45) Date of publication of patent specification: **11.09.91**　(51) Int. Cl.⁵: **B01L 3/02**

(21) Application number: **85302124.4**

(22) Date of filing: **27.03.85**

(54) Enclosed pipette tip rack.

(30) Priority: **14.09.84 US 650505**

(43) Date of publication of application:
**19.03.86 Bulletin 86/12**

(45) Publication of the grant of the patent:
**11.09.91 Bulletin 91/37**

(84) Designated Contracting States:
**AT BE CH DE FR GB IT LI LU NL SE**

(56) References cited:
**US-A- 3 937 322**
**US-A- 4 256 240**
**US-A- 4 349 109**
**US-A- 4 358 908**
**US-A- 4 436 700**

(73) Proprietor: **RAININ INSTRUMENT CO.,INC.**
**2200 Powell Street**
**Emeryville, CA 94608(US)**

(72) Inventor: **Rainin, Kenneth**
**26 Sea View**
**Piedmont, California 94611(US)**
Inventor: **Ruskewicz, Stephen**
**183 Ardmore Road**
**Kesington, California 94707(US)**

(74) Representative: **Barker, Rosemary Anne et al**
**MEWBURN ELLIS, Hollins Chambers, 64a**
**Bridge Street**
**Manchester M3 3BA(GB)**

**Description**

This invention relates to an enclosed pipette tip rack.

Disposable pipette tips have often been packaged in supporting trays. Such disposable pipette tip trays function to organize and to aid the user of the same in the placement of each disposable pipette tip on a pipette. Prior pipette tip trays generally had an open bottom and top. For example United States Patent 3,494,201 describes a tray or rack for disposable pipette tips and the system for use of the same.

Packages have also been devised to ship and store stackable pipette tip racks. Such packages are intended to protect the disposable pipette tips held therewithin from any type of contamination or physical damage. In this respect reference is made to United States Patent 3,853,217, to Scordato, United States Patent 3,937,322 to Cohen and French Patent 1,475,924, by way of example.

United States design patent D 271,239 issued to Lemieux et al. describes an individual container and rack for pipette tips which does not include the provision for nesting a plurality of the same as is depicted in the prior art, e.g. in United States Patent 3,853,271 to Scorado et al. Moreover, the container and rack for pipette tips shown in United States design patent D271,239 serves as a unitary package for disposable pipette tips and may be autoclaved. During autoclaving the top of the container and rack may be tilted to allow circulation of ambient gases to the interior of the container as as shown in a brochure issued by the assignee of United States Design patent D 271,239. Open racks of the prior art are often autoclaved in cardboard boxes or metal foil containers hand fashioned for that purpose.

These prior disposable pipette tips racks and containers and the techniques employed for autoclaving the same involve undesirable aspects. For example, the method of tilting the cover on the container and rack shown in United States design patent D 271,239 is unreliable since the top is difficult to balance in a tilted position and may shift from that position during an autoclaving process. In addition, the top must be repositioned after the autoclaving process to prevent contamination from dust, moisture, and other airborne contaminants. Using a secondary container for the autoclaving is cumbersome, expensive, and often results in contamination of the disposable pipette tip in the rack during the autoclaving process.

United States patent 4,358,908 describes a plant culture vessel. While the vessel includes a vent between a top of a container and a cover therefor, it does not include internal support for a pipette rack nor means for venting between chambers within the container formed by such a rack. Therefore, the venting provided by the plant culture vessel would be inadequate for any modification of the vessel to include an internal rack forming separate chambers within the vessel.

A disposable enclosed pipette tip rack which solves the problems encountered in the prior art, especially during the autoclaving process, would be a great advance in the scientific and medical field.

It is an object of the present invention to provide a pipette tip rack which is fully autoclavable and permits the autoclaving gas to freely contact the pipette tips within the enclosed rack.

It is yet another object of the present invention to provide a pipette tip rack which permits free communication between first and second chambers formed by a tray which holds the disposable pipette tips within the enclosed rack.

Another object of the present invention is to provide a pipette tip rack which may be steam autoclaved and which minimizes the condensation of steam in the vicinity of the disposable tips.

A further object of the present invention is to provide a pipette tip rack which requires a minimum of handling and therefore reduces the possibility of contamination of the pipette tips within the pipette tip rack.

In accordance with the present invention a novel and useful enclosed pipette tip rack is provided having the combination of features set forth in appended claim 1, with further optional features set forth in the subclaims.

The invention will be described further, by way of example, with reference to the accompanying drawings in which:

Fig. 1 a sectional view of a preferred practical embodiment of the pipette tip rack of the present invention showing a second rack in phantom to illustrate the stacking feature thereof;

Fig.2 is a sectional view taken along line 2-2 of Fig.1;

Fig. 3 is an enlarged top plan view of a pair of openings occupied by pipette tips of the tray portion of the pipette tip rack depicted in Fig. 2;

Fig. 4 is a sectional view taken along line 4-4 of Fig. 3 showing a broken side view of one pipette tip;

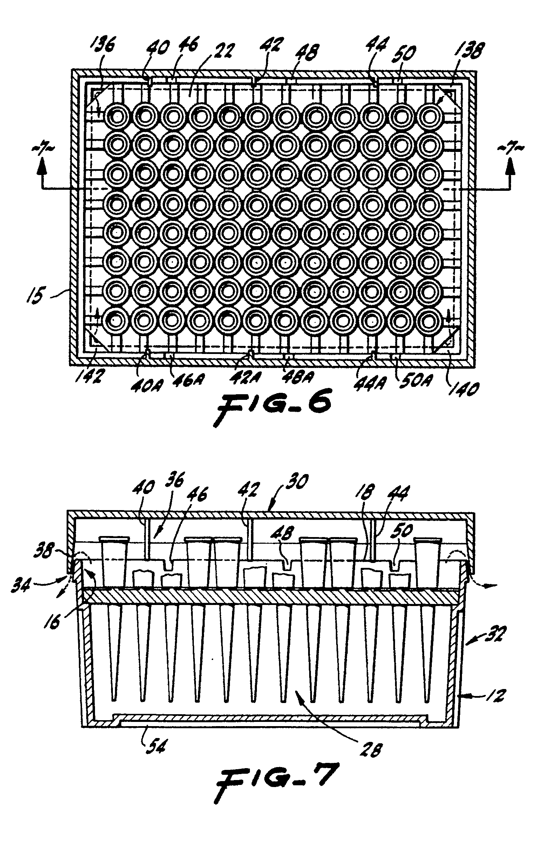

Fig. 5 is an enlarged partial sectional view showing the detail of the upper edge portion of the rack depicted in Fig. 1 along line 5-5 thereof;

Fig. 6 is a horizontal sectional view of the pipette tip rack shown in Fig. 1 emphasizing the tray portion within the rack;

Fig. 7 is a view taken along line 7-7 of Fig. 6 showing the rack of Fig. 1 in the autoclavable mode;

Fig. 8 is a sectional view depicting ventilation means between the first and second chambers of another embodiment of the pipette tip rack of the invention used with a pipette tip having a purely conical shape; and

Fig. 9 is a view taken along line 12-12 of Fig. 8.

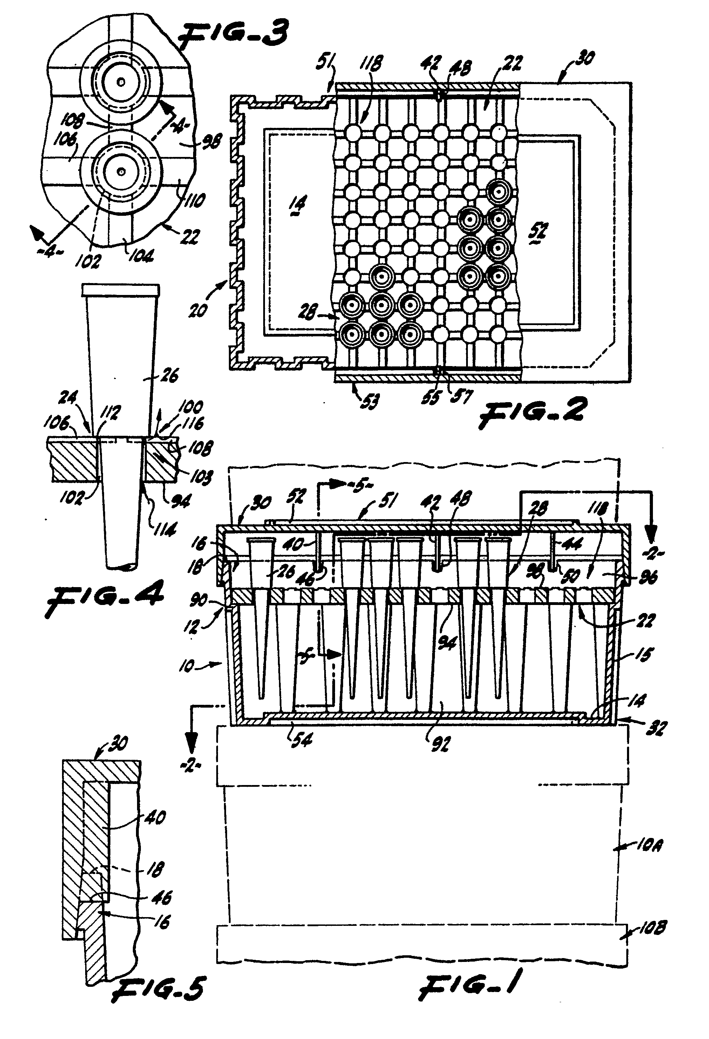

The pipette tip rack of the invention as a whole is indicated in the drawings by reference numeral 10. The pipette tip rack 10 includes a container 12, as shown in Fig. 1. The container 12 is constructed with a bottom 14 and a side wall 15 which, in the embodiment shown in Fig. 1, is continuous with the bottom 14 and extends upwardly therefrom. The side wall 15 terminates in a top portion 16 having a top surface 18 which extends completely around the container 12. Although the container 12 may take any shape, in the embodiments shown in Figs. 1 and 2, the container 2 has a rectangular configuration with a slightly tapered side wall 15. The side wall 15 may include a series of projections and recesses forming a generally fluted facade 20, as shown in Fig. 2, which strengthens the container 12 and confers aesthetic qualities thereto.

The pipette tip rack 10 also includes a tray 22, as shown in Figs. 1 and 2, which tray includes means 24 for holding at least one pipette tip 26. By way of illustration Fig. 1 depicts a plurality of pipette tips 28 which are located partially within the container 12.

The rack 10 also includes a cover 30 which is supported by the container 12 above and adjacent to the tray 22. The cover 30 and the container 12 form an enclosure 32, indicated in Fig. 1, which encases a plurality of pipette tips 28 held in the rack 22. The pipette tips 28 do not touch the container bottom 14 or the cover 30 within the enclosure 32. The container 12 and the cover 30 forming the enclosure 32. may be constructed of any rigid or semi-rigid material. However, plastics material such as polypropylene is preferred since it has been found that this material easily withstands a typical sterilization temperature of 132°C and a sterilization pressure of 1.9 kilograms per square centimeter. Also, polypropylene is resistant to chemical attack by sterilization gases such as steam, air, and ethylene oxide employed in autoclaving or other sterilization processes.

The enclosure 32 includes means 34 for venting the same. The means 34 is depicted in Fig. 7 as including means 36 for separating the cover 30 from the top portion 16 of the container 12. A gap 38 forms between the cover 30 and the top portion 16 when rib elements 40, 42, and 44 rest on the top surface 18, as shown in Fig. 7. When the cover 30 is positioned as shown in Fig. 7 (first position) the enclosure 32 includes the said means 34 for venting the enclosure 32. On the other hand, the cover 30 may be turned approximately 180° and assume the configuration shown in Fig. 1 (second position) where the rib elements 40, 42, and 44 fit within recesses 46, 48 and 50 along the sides of the top portion 16. Similar ribs and recesses may also be provided along the other side 53 of the top portion 16 (only rib 55 and recess 57 shown on Fig. 2). It should be apparent that the gap 38 is not present in this second position of the cover 30 relative to the container 12. In the absence of the gap 38, the enclosed rack 10 quite adequately resists intrusion of dust and other contaminants into the interior of the enclosure 32. This "dust-free" configuration is preferred for shipping and storing of the pipette tip rack 10.

The cover 30 also includes a raised portion 52 which fits into a depression or recess 54 in the bottom 14 of the container 12. Thus, racks 10, 10A and 10B may be stacked and partially nested during shipment, as indicated in Fig. 1.

Returning to Fig. 1 it may be seen that the tray 22 is supported by the container 12 on a shoulder 90 which extends around the interior of the enclosed pipette tip rack 10. The shoulder 90 is located at a height above the bottom 14 such that the pipette tips 28 do not touch the cover 30 or the bottom 14 of the enclosure 32. The tray 22 forms a first chamber 92 between the underside 94 thereof and the bottom 14 and a second chamber 96 between the upper side 98 thereof and the cover 30. Means 100 is provided for connecting the first chamber 92 to the second chamber 96. With reference to Figs. 3 and 4, it may be seen that the means 24 for holding each pipette tip 26 in the tray 22 comprises an opening 102 through the tray 22. Shoulders or plateaus 104, 106, 108 and 110 extend upwardly from the upper side 98 of the tray 22 and engage an annulus 112 of each pipette tip 126. Consequently a multiplicity of fluid passages 103 are formed between adjacent shoulders 104, 106, 108, 110 permitting communication between the first and second chambers 92 and 96 as per directional arrows 114 and 116 in Fig. 4. As depicted in Fig. 2, the tray 22 includes a plurality of openings 118 therethrough each capable of holding one of the plurality of pipette tips 28. A quartet of shoulders or plateaus is associated with each of the plurality of openings 118 to support each of the plurality of pipette tips 28 exemplified by the pipette tip 26 in Figs. 3 and 4.

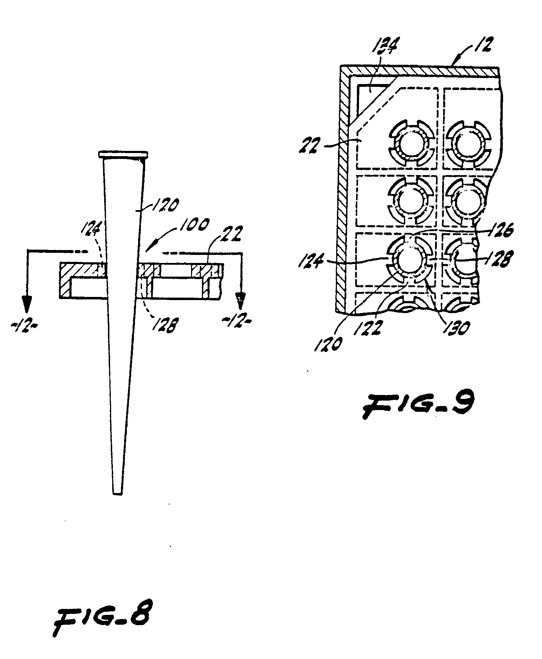

Figs. 8 and 9 illustrate an alternate embodiment of chamber connecting means 100 used in conjunction with a pipette tip 120 which does not include a shoulder. Protuberances 122, 124, 126 and 128 extend from the tray 22 to contact the exterior of each pipette tip 120. Thus, a plurality of passages 130 are formed between successive protuberances 124, 126 and 128 permitting fluids to pass from the first chamber 92 to the second chamber 96. In addition, the chambers 92 and 96 may be connected by vents 134 formed by mitering the corners of the tray 22, as shown in Fig. 9. Fig. 6 depicts the tray 22 having each corner mitred thus forming vents 136, 138, 140 and 142.

In operation, a user would load the pipette tips 28 into the openings 118 of the tray 22. The tray 22 would be placed within the container 12 and the cover 30 would be positioned on top of the container 12 in either the "dust-free" position, Fig. 1, or the autoclaving position, Figs. 6 and 7. In the latter position the pipette tip rack 10 is placed in a sterilizing or autoclaving environment and fluids are free to enter and exit the pipette tip rack 10 through the venting means 34. In addition, such fluids are free to move between enclosure chambers 92, 96 via means 100 which may include passages adjacent each pipette tip 28 or via the openings such as vents 136, 138, 140, and 142, shown in Fig. 6. After autoclaving, the cover 30 may be placed in the "dust-free" position to maintain the sterility of the pipette tips 28 within the rack 10.

The tray 22 may be removed from the enclosure 32 when the pipette tips 28 are no longer usable. At this junction, the cover 30 and the container 12 may form a case for any desired use.

## Claims

1. An enclosed pipette tip rack (10) comprising a container (12) having a bottom (14) and a side wall (15) connected to and extending from and around the perimeter of the bottom and terminating in a top portion (16), a sole tray (22) including means (24) for holding at least one pipette tip (26, 28), the container (12) further including means (90) supporting the tray (22) such that at least a portion of the at least one pipette (26) lies within the container, and a cover (30) supported by the container (12) adjacent the tray (22), the cover and container forming an enclosure (32) for the at least one pipette tip, the tray (22) forming a first chamber (92) between one side thereof and the container (12) and a second chamber (96) between another side thereof and the cover (30), characterised by provision of venting means (34) permitting circulation between the inside and outside of the enclosure (32) and between the first and second chambers (92, 96), the venting means (34) including means (36) for separating the cover (30) from the top portion (16) of the side wall (15) of the container (12) to form a gap (38), therebetween and further including means (100) joining the first chamber (92) to the second chamber (96) defining spaces (103, 130, 134, 136, 138, 140 or 142) to permit circulation of fluid between the first and second chambers (92, 96) other than through any pipette tip (26, 28) or through the holding means (24) devoid of any pipette tip.

2. A pipette tip rack according to claim 1 in which the means (36) for separating the cover (30) from the top portion (16) of the side portion (15) includes at least one element (40, 42, 44) connected to the cover (30) which can bear on the relevant side wall (15) and thereby form the gap (38) between the top portion (16) of that side wall (15) and the cover (30).

3. A pipette tip rack according to claim 1 or 2 in which the means (36) for separating the cover (30) from the top portion (16) of the side wall (15) includes at least one element connected to the container (12) which can bear on the cover (30) and thereby form the gap (38) between the top portion (16) of the side wall (15) and the cover (30).

4. A pipette tip rack according to claim 2 in which the container top portion (16) includes a surface (18) and the cover (30) is capable of being positioned on the container (12) in a first position in which each cover element (40, 42, 44) is supported by said surface (18) to form the gap (38) or in a second position such that the cover (30) engages the container top portion (16) and each cover element (40, 42, 44) is free of support by the surface (18) thereby substantially eliminating the said gap (38) between the container top portion (16) and the cover (30).

5. A pipette tip rack according to any preceding claim in which the means (24) for holding each pipette tip (26, 28) comprises an opening (102) through the tray (22) and the means (100) for connecting the first and second chambers (92, 96) includes a shoulder (104, 106, 108, 110) connected to the said other side of the tray (22) which shoulder (104, 106, 108, 110) engages the pipette tip (26, 28) to form a space or passageway (103) between the exterior of the pipette tip (26, 28) and the portion of the tray (22) surrounding the opening (102) through the tray (22).

6. A pipette tip rack according to any of claims 1 to 4 in which the means (24) for holding each pipette tip (120) comprises an opening through the tray (22) and the means (100) for connecting the first chamber (92) with the second chamber (96) includes a protuberance (122, 124, 126, 128) extending from the tray (22) into the opening (102) therethrough, said protuberance contacting the exterior of the pipette tip (120) to form a space or passage (130) between the exterior of the pipette tip (120) and the tray (22).

7. A pipette tip rack according to any preceding claim, in which the means (100) for connecting the first and second chambers (92, 96) includes an opening or vent (136, 138, 140, 142) formed at one corner of the tray (22).

8. A pipette tip rack according to any preceding claim in which the cover (30) includes a raised portion (52) and the bottom (14) of the container (12) includes a depression (54) which is sized to fit the raised portion (52) of the cover (30) during stacking of such racks.

9. A pipette tip rack according to any preceding claim in which the tray (22) is removable from the container (12).

**Revendications**

1. Réceptacle-support pour embout de pipette fermé (10) comportant un conteneur (12) ayant un fond (14) et une paroi latérale (15) reliée à et s'étendant depuis et autour du périmètre du fond et se terminant en une partie supérieure (16), un plateau de support (22) comprenant des moyens (24) destinés à maintenir au moins un embout de pipette (26, 28), le conteneur (12) comportant en outre des moyens (90) supportant le plateau (22) de telle sorte que au moins une partie de au moins une pipette (26) s'étende à l'intérieur du conteneur, et un couvercle (30) supporté par le conteneur (12) de manière adjacente au plateau (22), le couvercle et le conteneur formant une enceinte (32) pour l'embout de pipette, le plateau (22) formant une première chambre (92) entre un côté de celui-ci et le conteneur (12) et une deuxième chambre (96) entre un autre côté de celui-ci et le couvercle (30), caractérisé par le fait de prévoir des moyens de mise à l'air libre (34) permettant la circulation entre l'intérieur et l'extérieur de l'enceinte (32) et entre les première et deuxième chambres (92, 96), les moyens de mise à l'air libre (34) comportant des moyens (36) destinés à séparer le couvercle (30) de la partie supérieure (16) de la paroi latérale (15) du conteneur (12) afin de former entre eux un espace (38), et comprenant en outre des moyens (100) reliant la première chambre (92) à la deuxième chambre (96) en définissant des espaces (103, 130, 134, 136, 138, 140 ou 142) afin de permettre la circulation de fluide entre les première et deuxième chambres (92, 96) autrement que par les embouts de pipette (26, 28) ou par les moyens de support (24) vides de tout embout de pipette.

2. Réceptacle-support pour embout de pipette selon la revendication 1, dans lequel les moyens (36) destinés à séparer le couvercle (30) de la partie supérieure (16) de la partie latérale (15) comprennent au moins un élément (40, 42, 44) relié au couvercle (30) qui peut porter sur la paroi latérale concernée (15) et former ainsi l'espace (38) entre la partie supérieure (16) de cette paroi latérale (15) et le couvercle (30).

3. Réceptacle-support pour embout de pipette selon la revendication 1 ou 2, dans lequel les moyens (36) destinés à séparer le couvercle (30) de la partie supérieure (16) de la partie latérale (15) comprennent au moins un élément relié au conteneur (12) qui peut porter sur le couvercle (30) et former ainsi l'espace (38) entre la partie supérieure (16) de la paroi latérale (15) et le couvercle (30).

4. Réceptacle-support pour embout de pipette selon la revendication 2, dans lequel la partie supérieure du conteneur (16) comprend une surface (18) et le couvercle (30) peut être positionné sur le conteneur (12) dans une première position dans laquelle chaque élément de couvercle (40, 42, 44) est supporté par la dite surface (18) afin de former l'espace (38) ou dans une deuxième position de telle sorte que le couvercle (30) engage la partie supérieure du conteneur (16) et que chaque élément de couvercle (40, 42, 44) est dégagé du support par la surface (18), éliminant ainsi substantiellement le dit espace (38) entre la partie supérieure du conteneur (16) et le couvercle (30).

5. Réceptacle-support pour embout de pipette selon l'une quelconque des revendications précédentes, dans lequel les moyens (24) destinés à maintenir chaque embout de pipette (26, 28) comporte une ouverture (102) au travers du plateau (22) et les moyens (100) destinés à relier les première et deuxième chambres (92, 96) comprennent un épaulement (104, 106, 108, 110) relié au dit autre côté du plateau (22), lequel épaulement (104, 106, 108, 110) engage l'embout de pipette (26, 28) afin de former un espace ou passage (103) entre l'extérieur de l'embout de pipette (26, 28) et la partie du plateau (22) entourant l'ouverture (102) au travers du plateau (22).

**6.** Réceptacle-support pour embout de pipette selon l'une quelconque des revendications précédentes, dans lequel les moyens (24) destinés à maintenir chaque embout de pipette (120) comporte une ouverture au travers du plateau (22) et les moyens (100) destinés à relier la première chambre (92) à la deuxième chambre (96) comprennent une protubérance (122, 124, 126, 128) s'étendant depuis le plateau (22) et l'ouverture (102) au travers de celui-ci, la dite protubérance entrant en contact avec l'extérieur de l'embout de pipette (120) afin de former un espace entre l'extérieur de l'embout de pipette (120) et le plateau (22).

**7.** Réceptacle-support pour embout de pipette selon l'une quelconque des revendications précédentes, dans lequel les moyens (100) destinés à relier les première et deuxième chambres (92, 96) comprennent une ouverture ou évent (136, 138, 140, 142) formé au niveau d'un coin du plateau (22).

**8.** Réceptacle-support pour embout de pipette selon l'une quelconque des revendications précédentes, dans lequel le couvercle (30) comprend une partie relevée (52) et le fond (14) du conteneur (12) comprend une dépression (54) qui est dimensionnée pour recevoir la partie relevée (52) du couvercle (30) au cours de l'empilage de tels réceptacles-supports.

**9.** Réceptacle-support pour embout de pipette selon l'une quelconque des revendications précédentes, dans lequel le plateau (22) est amovible du conteneur (12).

## Patentansprüche

**1.** Geschlossener Pipettenspitzenhalter (10), mit einem Behälter (12), der einen Boden (14) und eine mit ihm verbundene und sich über den ganzen Umfang des Bodens erstreckende Seitenwand (15), die in einem oberen Randbereich (16) endet, aufweist, mit einem einzigen Einsatz (22) mit Mitteln (24) zum Halten von mindestens einer Pipettenspitze (26, 28), und bei welchem der Behälter (12) außerdem Mittel (90) zum Halten des Einsatzes (22) in einer Lage aufweist, daß mindestens ein Teil der mindestens einen Pipettenspitze (26) innerhalb des Behälters liegt, und mit einem vom Behälter (12) in der Nähe des Einsatzes (22) getragenen Deckel (30), wobei Deckel und Behälter eine Umhüllung (32) für die mindestens eine Pipettenspitze bilden und der Einsatz (22) eine erste Kammer (22) zwischen seiner einen Seite und dem Behälter (12) und eine zweite Kammer (96) zwischen seiner anderen Seite und dem Deckel (30) begrenzt, dadurch gekennzeichnet, daß Belüftungsmittel (34) vorgesehen sind, die eine Zirkulation zwischen dem Innern und der Außenseite der Umhüllung (32) und zwischen der ersten und der zweiten Kammer (92, 96) erlauben, und daß die Belüftungsmittel (34) Teile (36) zum Abhalten des Deckels (30) von dem oberen Randbereich (16) der Seitenwand (15) des Behälters (12) zur Ausbildung eines Spaltes (38) zwischen ihnen umfassen, und außerdem Mittel (100) zum Verbinden der ersten Kammer (92) mit der zweiten Kammer (96) vorgesehen sind, die Freiräume (103, 130, 134, 136, 138, 140 oder 142) bilden, die eine Flüssigkeitszirkulation zwischen der ersten und der zweiten Kammer (92, 96) erlauben, die nicht durch eine Pipettenspitze (26, 28) oder durch die für jede Pipettenspitze vorgesehenen Halter (24) hindurchführt.

**2.** Pipettenspitzenhalter nach Anspruch 1, in welchem die Teile (36) zum Abhalten des Deckels (30) vom oberen Randbereich (16) der Seitenwand (15) mindestens ein mit dem Deckel verbundenes Element (40, 42, 44) aufweisen, das auf der Seitenwand (15) aufstehen kann und dadurch den Spalt (38) zwischen dem oberen Randbereich (16) der Seitenwand (15) und dem Deckel (30) bildet.

**3.** Pipettenspitzenhalter nach Anspruch 1 oder 2, bei welchem die Teile (36) zum Abhalten des Deckels (30) von dem oberen Randbereich (16) der Seitenwand (15) mindestens ein mit dem Behälter (12) verbundenes Element aufweisen, das gegen den Deckel (30) anliegen kann und dadurch den Spalt (38) zwischen dem oberen Randbereich (16) der Seitenwand (15) und dem Deckel (30) bildet.

**4.** Pipettenspitzenhalter nach Anspruch 2, bei welchem der obere Randbereich (16) des Behälters eine Fläche (18) aufweist und der Deckel (30) auf dem Behälter (12) in einer ersten Stellung anordenbar ist, in welcher jedes Deckelelement (40, 42, 44) auf dieser Fläche (18) aufliegt, um den Spalt (38) zu bilden, oder in einer zweiten Stellung so anordenbar ist, daß der Deckel (30) den oberen Randbereich (16) des Containers umfaßt und jedes Deckelelement (40, 42, 44) ohne Anlage gegen die Fläche (18) bleibt und dadurch den Spalt (38) zwischen dem oberen Randbereich (16) des Behälters und dem Deckel (30) verhindert.

**5.** Pipettenspitzenhalter nach einem der vorangehenden Ansprüche, bei welchem die Mittel (24) zum Halten jeder Pipettenspitze (26, 28) eine Durchgangsöffnung (102) im Einsatz (22) aufweisen, und die Mittel (100) zum Verbinden der ersten und zweiten Kammer (92, 96) eine mit der anderen Seite des Einsatzes (22) verbundene Schulter (104, 106, 108, 110) aufweisen, welche die Pipettenspitze (26, 28) so abstützt, daß ein Durchgangskanal (103) zwischen der Außenseite der Pipettenspitze (26, 28) und dem die Öffnung (102) des Einsatzes (22) umgebenden Teil des Einsatzes (22) entsteht.

6. Pipettenspitzenhalter nach einem der Ansprüche 1 bis 4, bei welchem die Mittel (24) zum Halten jeder Pipettenspitze (120) eine Durchgangsöffnung im Einsatz (22) aufweisen, und die Mittel (100) zum Verbinden der ersten Kammer (92) mit der zweiten Kammer (96) einen vom Einsatz (22) in die Durchgangsöffnung (102) abstehenden Vorsprung (122, 124, 126, 128) aufweisen und dieser Vorsprung die Außenseite einer Pipettenspitze (120) so berührt, daß ein Abstand oder Durchgang (130) zwischen der Außenseite der pipettenspitze (120) und dem Einsatz (22) gebildet ist.

7. Pipettenspitzenhalter nach einem der vorangehenden Ansprüche, bei welchem die Mittel (100) zum Verbinden der ersten und zweiten Kammer (92, 96) eine Öffnung oder einen Durchgang (136, 138, 140, 142) aufweisen, der an einer Ecke des Einsatzes (22) gebildet ist.

8. Pipettenspitzenhalter nach einem der vorangehenden Ansprüche, bei welchem der Deckel (30) einen erhöhten Teil (52) und der Boden (14) des Behälters (12) eine Ausnehmung (54), in welche beim Stapeln der Pipettenhalter der erhöhte Teil (52) des Deckels (30) paßt, aufweisen.

9. Pipettenspitzenhalter nach einem der vorangehenden Ansprüche, bei welchem der Einsatz (22) aus dem Behälter (12) entfernbar ist.

FIG-3

FIG-2

FIG-4

FIG-5

FIG-1

FIG-6

FIG-7

FIG_8

FIG_9